# EUROPEAN PATENT APPLICATION

(11) **EP 2 559 952 A1**
(43) Date of publication of application: **20.02.2013**
(21) Application number: 11768701.2
(22) Date of filing: 23.03.2011
(51) Int. Cl.: F24F 7/00, B01D 46/44, B60H 3/06

(54) **AIR PURIFIER**

(30) Priority: 15.04.2010 JP 2010094394
(71) Applicant: Sharp Kabushiki Kaisha, Osaka-shi, Osaka 545-8522 (JP)
(72) Inventor: MATSUBARA, Yoshihiko, Osaka-shi, Osaka 545-8522 (JP)
(74) Representative: Treeby, Philip David William
(86) International application number: PCT/JP2011/056954
(87) International publication number: WO 2011/129180

(57) **Abstract**

An air purifier is provided which can effectively purify the air for a long period of time without increasing the capacity of an internal power supply and an external power supply. A control section 51 determines, by means of a power supply detection circuit 57, whether or not the external power supplies power. The control section 51 causes an ion generator drive circuit 53 and a fan motor drive circuit 54 to perform operations in accordance with the determination. For example, the control section 51 causes the ion generator drive circuit 53 and the fan motor drive circuit 54 to perform continuous operation in a case where the external power supply supplies power, and causes them to perform intermittent operation in a case where the external power supply does not supply power.

## Description

### Technical Field

The present invention relates to an air purifier for purifying air. More particularly, the present invention relates to an air purifier such as an ion generating apparatus in which power is supplied by an internal power supply and/or an external power supply.

### Background Art

Room air includes fine particles and/or gas that are harmful to human body such as dusts, pollens, or tobacco smoke. In recent years, as a room becomes more airtight, more of these fine particles and/or gas remain in a room. At the same time, ventilation for preventing the fine particles and/or gas from remaining in the room is performed less frequently. In such circumstances, an ion generating apparatus having a function for purifying room air has become popular.
The above circumstances are not limited to a residential room. It also adapts to an interior of a vehicle.

The ion generating apparatus takes in room air by rotation of an air blower, mixes ions released by an ion generator into the air taken in, and blows out the air including the ions from a blowing port into a room. The ions included in the blown out air purifies air by sterilizing airborne bacteria and the like or charging dusts and the like.

For an air purifier for purifying the room air in a vehicle, an effect of purifying the air by the air purifier varies depending on a power source status of the vehicle and a vehicle condition such as whether or not the vehicle is operating or there are any persons in the vehicle. Patent Documents 1 and 2 disclose an air purifier that performs operation according to a vehicle condition and a method of operating the air purifier, respectively.

Patent Document 1 describes an air purifying device for a vehicle capable of enhancing functions and effects of a plasma cluster when cleaning an interior of the vehicle. When there are many passengers, effects of bacterial elimination, deodorization and refreshment are small. Thus, this air purifying device for the vehicle determines whether or not the vehicle is operating, and generates ions into the air when the air purifying device determines that the vehicle is parked, that is, when it is considered to be a few passengers.

Patent Document 2 describes an method of operating an air purifier for a vehicle which immediately stops operation of an electric air blower when rotation of an engine stops even if a ripple generated by the electric air blower becomes greater, to reduce consumption of a battery. With this operating method, the air is not purified when rotation of the engine is stopped and the ripple is not generated in a power source, whereas the air is purified by turning on and off of the operation repeatedly at regular intervals when the engine is rotated.

### Prior Art Document

### Patent Document

Patent Document 1: Japanese Patent Application Laid-Open No. 2005-75233
Patent Document 2: Japanese Patent Application Laid-Open No. 3-10925 (1991)

### Disclosure of the Invention

### Problems to be Solved by the Invention

Today, air purifiers for purifying room air in a vehicle are available, which are supplied with power by an external power supply such as an in-vehicle cigar lighter socket or an in-vehicle circuit, and by a rechargeable internal power supply. The former, however, operates only when the vehicle is operating, and the latter cannot be used continuously for a long period of time. An air purifier having the internal power supply and also being supplied with power by an external power supply needs to be used for a longer time in a more effective manner.

In a case where the air purifier is used for a long period of time while a vehicle is parked, it is more likely that no passenger is inside the vehicle. In addition, the room in a vehicle is a closed small space. Thus, the room in the parked vehicle is hard to be contaminated once it is purified. Therefore, even if the air purifier is continuously used while the vehicle is parked, the effect reaches its peak over time and becomes less efficient.

The present invention has been contrived in view of the above circumstances. A main object of the invention is to provide an air purifier which can effectively purify the air for a long period of time without increasing the capacity of an internal power supply and that of an external power supply.

### Means for Solving the Problems

The air purifier according to the present invention performs an operation for purifying air by means of an internal power supply and/or an external power supply. The air purifier includes: a determining section for determining whether or not power is supplied by the external power supply; and a control section for causing the air purifier to perform the operation with different power consumption based on a determination made by the determining section.

The air purifier according to the present invention including an internal power supply and also being supplied with power by the external power supply, purifies the air by these power supplies. The air purifier also includes the determining section and the control section. The determining section determines whether or not the power is supplied by the external power supply. The control section causes the air purifier to perform the operation with different power consumption such as a continuous operation or an intermittent operation based on a determination made by the determining section.
For example, the control section causes the air purifier to perform the intermittent operation with low power consumption in the case where power is not supplied by the external power supply, that is, where power is supplied by only the internal power supply, and causes the air purifier to perform the continuous operation with high power consumption in the case where power is supplied by the external power supply. This operational switching is effective in an environment where it is hard to be contaminated in the case where the external power supply does not supply power, and where it is easy to be contaminated in the case where the external power supply supplies power. Here, the air can effectively be purified for a long period of time without increasing the capacity of the internal power supply and that of the external power supply.

In the air purifier according to the present invention, the internal power supply is chargeable by the external power supply.

For the air purifier according to the present invention, the internal power supply is chargeable by the external power supply in the case where power is supplied by the external power supply.
Therefore, the air can be purified only by the internal power supply for a longer period.

In the air purifier according to the present invention, the control section causes the air purifier to perform: an intermittent operation in a case where power is not supplied by the external power supply; and a continuous operation or an intermittent operation with a duty ratio greater than a duty ratio of the intermittent operation in a case where the power is supplied by the external power supply.

For the air purifier according to the present invention, the control section causes the air purifier to perform an intermittent operation in the case where power is not supplied by the external power supply, that is, power is supplied by only the internal power supply. Moreover, in the case where power is supplied by the external power supply, the control section causes the air purifier to perform a continuous operation or an intermittent operation with a duty ratio greater than a duty ratio of the intermittent operation performed in the case where power is not supplied by the external power supply.
For example, in the interior of a vehicle, an external power supply such as a cigar lighter socket supplies power when the vehicle is ready to be driven. Since it is more likely that the interior of the vehicle is occupied when the vehicle is ready to be driven, the interior of the vehicle is easy to be contaminated. On the other hand, when the vehicle is not ready to be driven, it is more likely that the interior of the vehicle is closed and not occupied, and therefore, the interior of the vehicle is hard to be contaminated. Consequently, the control section causes the air purifier to perform the above-described operations in an environment such as the interior of the vehicle so that the air can be purified for a longer time.

In the air purifier according to the present invention, the control section causes the air purifier to perform: an operation with low power consumption in a case where power is not supplied by the external power supply; and an operation with high consumption in a case where power is supplied by the external power supply.

For the air purifier according to the present invention, the control section causes the air purifier to perform the operation with low power consumption in the case where power is not supplied by the external power supply, that is, power is supplied by only the internal power supply. Moreover, the control section causes the air purifier to perform the operation with high power consumption in the case where power is supplied by the external power supply.
When the operation with higher power consumption is performed, the air is purified to be cleaner. Accordingly, in an environment such as the interior of the vehicle, where it is hard to be contaminated in the case where power is not supplied by the external power supply, and easy to be contaminated in the case where power is supplied by the external power supply, the air is maintained in a pure condition for a longer time.

The air purifier according to the present invention further includes an accepting section for accepting a selection of either of a continuous operation and an intermittent operation, wherein the control section causes the air purifier to perform an operation according to the selection accepted by the accepting section.

The air purifier according to the present invention further includes the accepting section. The accepting section accepts a selection of either of the continuous operation and the intermittent operation. The control section causes the air purifier to perform the operation according to the selection accepted by the accepting section.
The accepting section accepts the selection of either of the continuous operation and the intermittent operation from the user. The user judges how much the air is contaminated and can cause the air purifier to perform the operation as he/she desires.

### Effects of the Invention

According to the present invention, an air purifier which can effectively purify air for a long period of time without increasing the capacity of the internal power supply and that of the external power supply can be realized.

### Brief Description of the Drawings

FIG. 1 is a cross-sectional side view illustrating a substantial portion of an air purifier according to the present embodiment.
FIG. 2 is an explanatory view for explaining contents of operation of the air purifier, which are displayed on an operation panel.
FIG. 3 is a block diagram illustrating a substantial portion of the air purifier.
FIG. 4 is a circuit diagram illustrating a substantial portion of a power supply detection circuit.
FIG. 5 is a flowchart illustrating a processing procedure executed by a control section.
FIG. 6 is a flowchart illustrating a processing procedure executed by the control section.
FIG. 7 is a timing chart illustrating a timing of turning on/off of four types of intermittent operations.
FIG. 8 is an illustrative table illustrating an example of a power supply used in accordance with each combination of contents of operation and wind power of an air blower.

### Best Mode for Carrying Out the Invention

The present invention will be described in detail based on the drawings illustrating an embodiment thereof. Moreover, the present invention will be described with an example of an air purifier 1 installed in an interior of a vehicle.

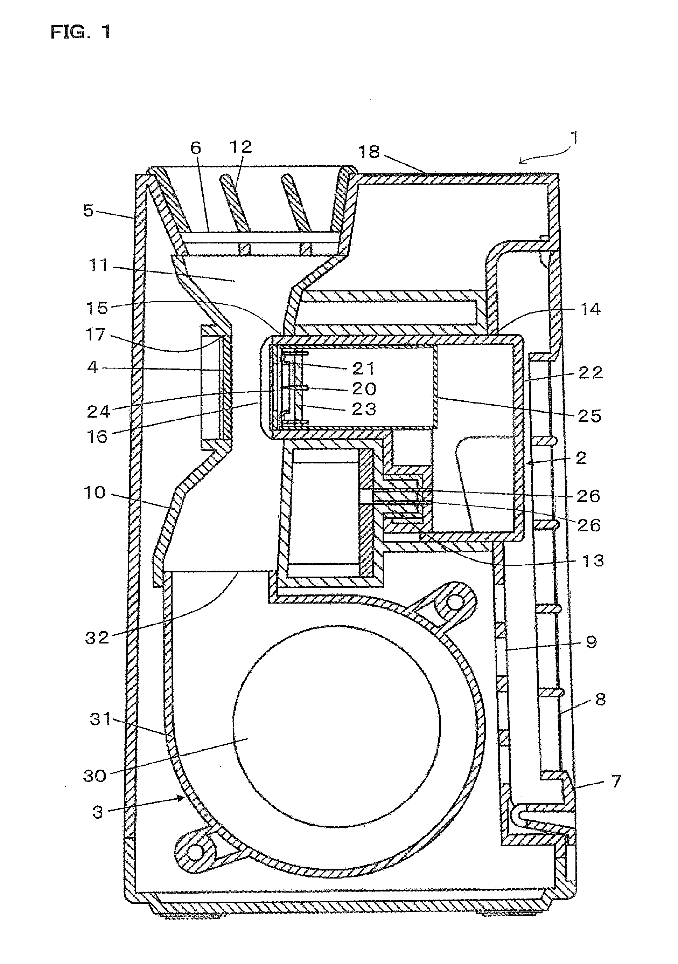

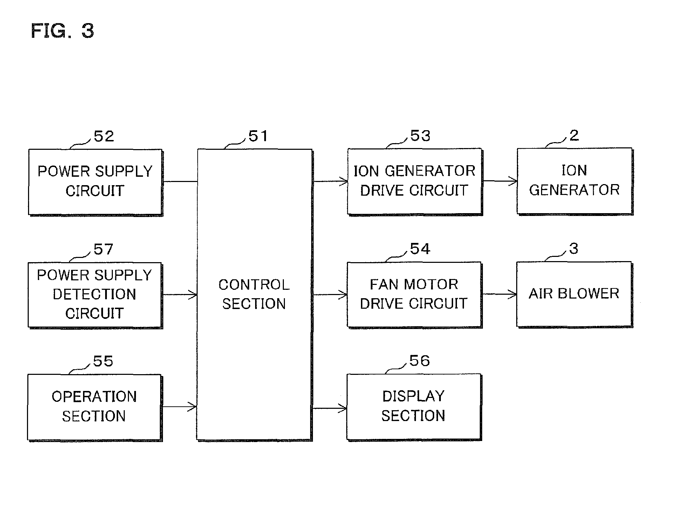

FIG. 1 is a cross-sectional side view illustrating a substantial portion of the air purifier 1 according to the present embodiment. The air purifier 1 includes an ion generator 2 for generating ions, an air blower 3 for blowing out the generated ions, and an ion detector 4 for detecting the generated ions. These are contained in a body case 5 forming a hollow rectangular parallelepiped shape. The air purifier 1 further includes a control section 51 described later (see FIG. 3). The control section 51 consists of a microcomputer to control drive of the ion generator 2 and the air blower 3. Also, the control section 51 causes the ion detector 4 to detect ions and determines whether or not the ions are generated.

On one end surface of the body case 5, a blowing port 6 having a rectangular shape is formed near one side surface, while, a cover 7 having a rectangular shape is provided in a detachable manner on another side surface opposite to that side surface. The cover 7 is provided with a suction port 8 with a filter and also has an air ventilation hole of a stripe pattern in a direction perpendicular to the longitudinal direction of the side surface. A suction port 9 having the same shape as the suction port 8 is provided behind the suction port 8 (in a direction perpendicular to the side surface having the cover 7 and toward the inside of the body case 5) near the end surface opposed to the end surface on which the blowing port 6 is placed. The air blower 3 having a cylindrical shape is provided further behind the suction port 9 (in a direction perpendicular to the side surface having the cover 7 and toward the inside of the body case 5).

A duct 10, having a rectangular cylinder shape, the longitudinal direction of which is along the side surface, is provided between the air blower 3 and the blowing port 6. Inside of the duct 10, an air path 11, having the same shape as the duct 10, is provided, and wind sent out from the air blower 3 is blown out from the blowing port 6 through the air path 11. The duct 10 has a structure that is widened at the blowing port 6 side and the air blower 3 side, and is narrowed at the middle part. The duct 10 is in communication with the blowing port 6.

The blowing port 6 is provided with a louver 12 having a rectangular shape in a detachable manner. A generation window 15 and a detection window 17 each having a rectangular shape are provided, at the middle part of the duct 10 where the air path 11 is the most narrowed, in a direction perpendicular to the side surface having the cover 7. The ion generator 2 having a box shape and the ion detector 4 having a plate shape are fit into the generation window 15 and the detection window 17 respectively. One surface of the ion generator 2 and one surface of the ion detector 4 face each other across the air path 11. Fitting the ion generator 2 and the ion detector 4 to the narrowest middle part of the air path 11 in the duct 10 allows effective use of a space in the body case 5. Thus, the air purifier 1 can be reduced in size.

The duct 10 is also joined to the air blower 3. Wind sent from the air blower 3 passes through the duct 10 and is blown out from the blowing port 6. The air blower 3 includes a fan 30 and a fan casing 31. The fan 30 is a sirocco fan having blades. The fan casing 31 is a cylindrical-shaped case that houses the fan 30 in a rotatable manner, and the fan casing 31 is installed in the body case 5. The fan 30 is rotated by a fan motor (not shown). For the fan casing 31, a rectangular-shaped blowing port 32 is formed at a place opposite to the blowing port 6 and is joined to the duct 10 to be in communication with the air path 11.

The air suctioned by the air blower 3 from the suction ports 8 and 9 is blown out from the blowing port 32 to the air path 11 in the duct 10. The air blown out from the blowing port 32 includes ions generated by the ion generator 2 at the middle part of the duct 10 to be blown out from the blowing port 6. The wind passing through the duct 10 flows from the blowing port 32 to the blowing port 6.

The ion generator 2 includes a discharge electrode 20, a dielectric electrode 21, and a storing case 22 of a box shape which contains the discharge electrode 20 and the dielectric electrode 21. One surface of the storing case 22 faces the air path 11 in the duct 10 and the discharge electrode 20 and the dielectric electrode 21 are formed on that surface. An external shape of the storing case 22 is the same as the external shape of the ion generator 2. The discharge electrode 20 is a needle electrode and the dielectric electrode 21 forms a ring shape. The discharge electrode 20 is arranged at the center of the dielectric electrode 21.

Two sets of the discharge electrodes 20 and the dielectric electrodes 21 are arranged in a direction parallel to the end surface on which the blowing port 6 and to the side surface on which the cover 7 is set. Moreover, the discharge electrodes 20, 20 and the dielectric electrodes 21, 21 are supported by a supporting substrate 23 having a rectangular shape. The supporting substrate 23 is placed in parallel to the side surface having the cover 7, and is contained in the storing case 22 near one surface thereof facing the air path 11. One of the discharge electrodes 20, 20 generates positive ions and the other discharge electrode 20 generates negative ions.

Two penetration holes 24, 24 of a circular shape are provided with one surface of the storing case 22 facing the air path 11. The penetration hole 24 and the dielectric electrode 21 face each other and the diameter of the penetration hole 24 is about the same as that of the dielectric electrode 21. Each of the discharge electrodes 20, 20 are positioned at the center of each of the penetration holes 24, 24. The storing case 22 is provided with a high voltage generating circuit (not shown) that applies a high voltage to the respective discharge electrodes 20, 20. The high voltage generating circuit is connected to the control section 51.

The discharge electrodes 20, 20, the dielectric electrodes 21, 21 and the high voltage generating circuit are unitized as an ion generating unit to be stored in a storing case 25 of a box shape. The storing case 25 is fit in the storing case 22 in a detachable manner. On a surface of the storing case 22 facing the duct 10 near the air blower 3, two pin connectors 26, 26 of a rectangular plate shape, each longitudinal direction of which is in parallel to the end surface of the body case 5, are placed to project in the direction of the duct 10. The pin connectors 26, 26 are inserted in a socket 13 of a box shape. The control section 51 outputs a drive signal to the high voltage generating circuit through the pin connectors 26, 26. Moreover, a direct-current power source or an alternating-current power source is supplied to the high voltage generating circuit through the pin connectors 26, 26.

The storing case 22 is placed in the body case 5 in a detachable manner. On a surface parallel to the side surface having the cover 7 at the blowing port 6 side of the surface on which the suction port 9 is formed, a rectangular shaped insertion opening 14 of the storing case 22 is formed in a direction perpendicular to the side surface having the cover 7. The storing case 22 is attached to and removed from the insertion opening 14 while the cover 7 is removed. The storing case 22 has a claw which is hooked to a resilient notch part (not shown) formed in the body case 5 when the storing case 22 is fit in the insertion opening 14. The generation window 15 is formed on the cover 7 side of the side surface of the air path 11, and the storing case 22 is fit in the generation window 15. The fit part of the storing case 22 is exposed to the air path 11.

The penetration holes 24, 24 are provided on one surface of the storing case 22 exposed to the air path 11. On the exterior of the storing case 22, an arched guard rib 16 is provided for each of the penetration holes 24, 24. The guard rib 16 covers the penetration hole 24, thereby preventing a risk that a user may directly touch the discharge electrode 20. When the storing case 22 is fit in the duct 10, the guard rib 16 projects into the air path 11 and is placed in parallel to the side surface having the cover 7.

When the storing case 22 is pulled out from the body case 5, the notch part of an fitting part for mounting (not shown) of the storing case 22 is deformed, then the claw is unhooked, and the storing case 22 can be taken out of the body case 5. The storing case 22 can be opened or closed. By opening the storing case 22, the storing case 25 can be taken out. In this way, the ion generator 2 can be used as a cartridge.
Therefore, in the case where the ion generator 2, for example, reaches the end of the product lifetime, the cartridge may be replaced. Also, by disassembling the cartridge and maintaining the ion generator 2, even the cartridge which reaches the end of product lifetime can be regenerated and the ion generator 2 can be reused.

The ion detector 4 includes a collecting body (not shown) for collecting the generated ions and an ion detection circuit (not shown) for outputting a detection signal corresponding to the collected ions to the control section 51. The conductive collecting body is a collecting electrode provided on a surface of a circuit board (not shown), which is exposed to the air path 11 , and is formed with a copper tape. The ion detection circuit is mounted on the backside of the circuit board on which the collecting electrode is formed. The ion detection circuit is electrically connected to the collecting body on the circuit board to be connected to the control section 51 through a lead wire.

The ion detection circuit consists of a diode for rectification and P-MOSFET (P-type Metal-Oxide Semiconductor Field-Effect Transistor) and the like (not shown). It is noted that the ion detection circuit is, for example, disclosed in Japanese Patent Application Laid-Open No. 2007-114177.
The ion detector 4 detects either positive ions or negative ions. When the collecting body collects the generated either positive or negative ions, the electrical potential of the collecting body is raised. The electrical potential of the collecting body is proportional to the amount of collected ions. The ion detection circuit performs A/D (Analog to Digital) conversion of an output voltage corresponding to this electrical potential, and then outputs a voltage obtained by A/D conversion to the control section 51. The control section 51 makes a determination regarding the generation of ions based on the voltage value input from the ion detector 4.

The ion detector 4 faces the air path 11 to be opposed to the guard rib 16. The duct 10 is provided with the detection window 17 in which the ion detector 4 is fit. The circuit board is fit in the detection window 17. The collecting body formed on the circuit board is exposed to the air path 11 to be opposed to the ion generator 2. The collecting body is opposed to one of the two discharge electrodes 20, 20, thereby intensively collecting ions generated from the opposing discharge electrode 20.

The ion generator 2 generates the positive ions and the negative ions from the two discharge electrodes 20, 20 respectively. Therefore, the ion detector 4 may collect both the positive ions and negative ions instead of collecting one of them.

For preventing this situation, the ion detector 4 is provided with a protecting body (not shown). The protecting body made of a metal plate is provided on a surface of the circuit board, which is exposed to the air path 11. The protecting body is placed opposed to the discharge electrode 20 which generates negative ions (or positive ions), the polarity of which is opposite to the collected positive ions (or the collected negative ions), and the protecting body expands radially toward the discharge electrode 20. The collecting body and protecting body are electrically isolated. The protecting body collects the negative ions (or the positive ions) generated from one of the discharge electrodes 20, 20 to protect the collecting body from the negative ions (or the positive ions). This prevents the collecting body from collecting the negative ions (or the positive ions).

The collecting body is larger than the protecting body and is opposed to the discharge electrode 20 that generates positive ions (or negative ions). This enables the positive ions (or the negative ions) to be intensively collected, and the precision for the ion detection to be higher. Furthermore, since the discharge electrode 20 is placed off-set from the center of the guard rib 16, the ion generation and dispersion are not interrupted. This enables the collecting body to certainly collect the generated positive ions (or the generated negative ions).

A distance between the ion generator 2 and the ion detector 4 is specified by a predetermined distance. The ions are generated from the discharge electrode 20 by corona discharge generated between the discharge electrode 20 and the dielectric electrode 21. The generated ions are emitted towards the ion detector 4 placed opposed to the ion generator 2, and the highly concentrated ions are radially distributed around tip of the discharge electrode 20.

When the tip of the discharge electrode 20 is too close to the ion detector 4 being opposed thereto (or a wall of the duct 10), discharge is generated between the tip of the discharge electrode 20 and the ion detector 4 (or a wall of the duct 10). This makes the discharge generated between the discharge electrode 20 and the dielectric electrode 21 unstable and thus the discharge won't be maintained. In order that the ion generator 2 generates ions stably, a predetermined distance, e.g. 10mm or more, is kept between the ion generator 2 and the ion detector 4. The narrowed middle part of the air path 11 in the duct 10 is designed corresponding to this distance.

By specifying the space between the ion generator 2 and the ion detector 4 as above, the ion generator 2 generates the ions stably. Also, ions of the highest concentration immediately after being generated from the ion generator 2 exist between the ion generator 2 and the ion detector 4. Accordingly, the ion detector 4 accurately detects the ion generation.

On the end surface on which the blowing port 6 is formed at the side of the side surface on which the cover 7 is formed, an operation panel 18 of a rectangular shape is provided as an accepting section described in Claims. The operation panel 18 includes an operation section 55 and a display section 56 (see FIG. 3) described later. The operation section 55 includes an operation switching button, a wind power switching button and so on. By pressing the operation switching button, the operation is switched to one of "AUTOMATIC", "CONTINUOUS", "INTERMITTENT" and "OFF." By pressing the wind power switching button, the wind power is switched to one of "HIGH", "MEDIUM" and "LOW." When the operation section 55 is operated, the control section 51 drives the ion generator 2 and the air blower 3 and at the same time causes the display section 56 to display text indicating that the air purifier 1 is operating.

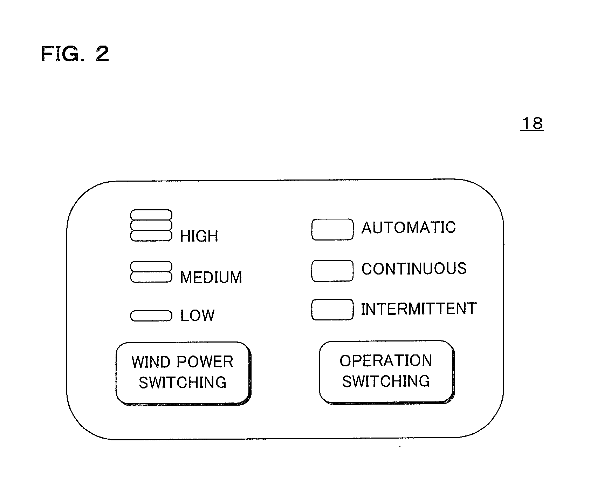

FIG. 2 is an explanatory view for explaining contents of operation of the air purifier 1 displayed on the operation panel 18. Three types of the operations, namely "AUTOMATIC", "CONTINUOUS" and "INTERMITTENT" are set for the air purifier 1, and any one of these operations is operated. As illustrated in FIG. 2, lights next to texts displayed as "AUTOMATIC", "CONTINUOUS" and "INTERMITTENT" are lit on the operation panel 18 based on the executed operation.

In addition, the "LOW", "MEDIUM" or "HIGH" wind power is set for the air purifier 1. The wind power is adjusted by a user's operation of the operation panel 18. As illustrated in FIG. 2, lights next to texts displayed as "LOW", "MEDIUM" and "HIGH" are lit on the operation panel 18 based on the output wind power.

When the air purifier 1 is operated, positive ions are generated from one of the discharge electrodes 20, 20 of the ion generator 2 whereas negative ions are generated from the other discharge electrode 20. The generated ions are carried on the wind blown out from the air blower 3 to be blown out to the outside from the blowing port 6. The blown out ions decompose airborne molds, viruses or the like floating inside a vehicle to removed them.

FIG. 3 is a block diagram illustrating a substantial portion of the air purifier 1. The control section 51 is connected to a power supply circuit 52, an ion generator drive circuit 53, a fan motor drive circuit 54, the operation section 55, the display section 56 and a power supply detection circuit 57. The power supply circuit 52 supplies power to the control section 51.

The ion generator drive circuit 53 drives the ion generator 2 and is controlled by the control section 51. The fan motor drive circuit 54 drives the fan of the air blower 3 and is controlled by the control section 51. The operation section 55 included in the operation panel 18 accepts a user's instruction for contents of operation, wind power or the like, and transmits the instruction to the control section 51. The display section 56 displays the contents of operation, wind power or the like performed by the air purifier 1, and is controlled by the control section 51.

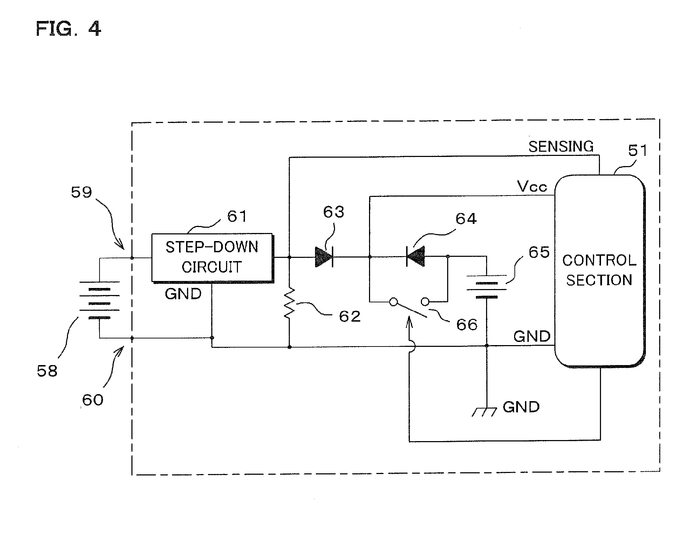

FIG. 4 is a circuit diagram illustrating a substantial portion of a power supply detection circuit 57. The air purifier 1 includes an internal power supply 65, an external power supply 58 and connection terminals 59, 60. The external power supply 58 is, for example, a cigar lighter socket in the vehicle. The power supply detection circuit 57, in which the connection terminals 59, 60 are connected to the external power supply 58, detects whether or not the external power supply 58 supplies power to the control section 51. A positive terminal and a negative terminal of the external power supply 58 are connected to the connection terminals 59 and 60 respectively. The connection terminal 60 is connected to a ground (hereinafter also referred to as "GND").

The control section 51 includes a sensing terminal for sensing whether or not the external power supply 58 supplies power. A step-down circuit 61 is connected between the sensing terminal of the control section 51 and the connection terminal 59. When the external power supply 58 is connected to the air purifier 1 to supply power, the step-down circuit 61 lowers a voltage (of 12 volts, for example) output from the external power supply 58 and outputs the lowered voltage (of 5 volts, for example) to the sensing terminal of the control section 51. In the case where the external power supply 58 does not supplies power, the sensing terminal of the control section 51 does not sense a voltage of the step-down circuit 61 because no voltage is applied to the connection terminal 59. A GND terminal of the step-down circuit 61 is connected to the GND.

A resistance 62 is connected between a terminal of the control section 51 side of the step-down circuit 61 and the GND. A diode 63 is connected between a Vcc terminal of the control section 51 and a connection node of the step-down circuit 61 and the resistance 62. An anode of the diode 63 is connected to the connection node of the step-down circuit 61 and the resistance 62, and a cathode of the diode 63 is connected to the Vcc terminal of the control section 51. The Vcc terminal is a terminal that accepts power supplied by the control section 51. When the external power supply 58 is connected to supply power, the voltage of the external power supply 58 lowered by the step-down circuit 61 is supplied to the Vcc terminal of the control section 51 through the diode 63.

A series circuit of a diode 64 and the internal power supply 65 is connected between a cathode of the diode 63 and the GND. The cathode of the diode 64 is connected to the cathode of the diode 63, an anode of the diode 64 is connected to a positive terminal of the internal power supply 65, and a negative terminal of the internal power supply 65 is connected to the GND. The voltage (of 5 volts, for example) of the internal power supply 65 is applied to the Vcc terminal of the control section 51 through the diode 64. The GND terminal of the control section 51 is connected to the GND. Therefore, power is supplied to the air purifier 1 by the internal power supply 65 and the external power supply 58. Also, only the internal power supply 65 supplies power in the case where the external power supply 58 does not supply power, and only the external power supply 58 supplies power in the case where the internal power supply 65 does not supply power.
Moreover, a switch 66 is connected in parallel to the diode 64, and the control section 51 controls on/off of the switch 66.

The control section 51, which is a determining section and a control section described in Claims, determines whether or not the power is supplied from the external power supply 58 based on whether or not the voltage of the external power supply 58 lowered by the step-down circuit 61 is sensed at the sensing terminal. Also, the control section 51 turns on the switch 66, when the control section 51 determines that the external power supply 58 supplies power. The internal power supply 65 is supplied with a voltage of the external power supply 58 lowered by the step-down circuit 61 and is charged. As just described, the internal power supply 65 is chargeable by the external power supply 58. The control section 51 turns off the switch 66 when the control section 51 determines that the external power supply 58 does not supply power.
Also, the external power supply 58 and/or the internal power supply 65 supplies power to the ion generator 2, the air blower 3, the display section 56 and the like through the control section 51.

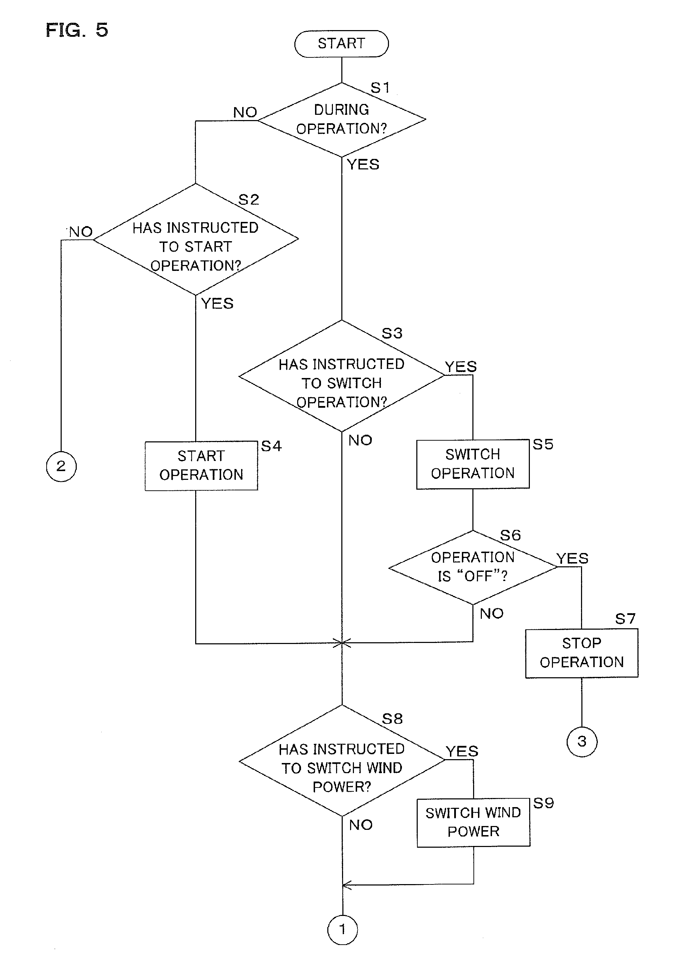

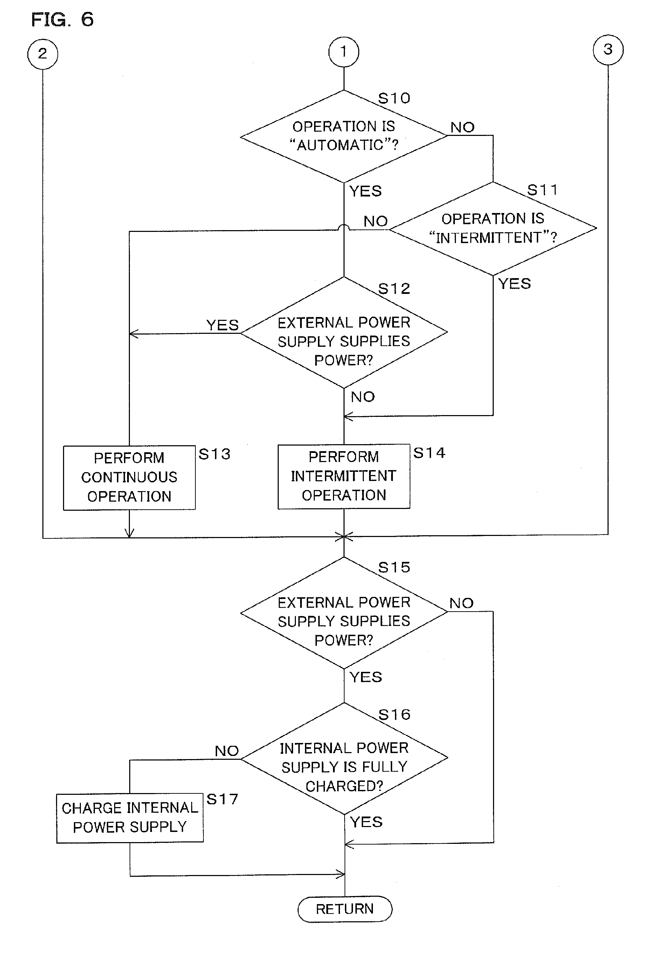

FIGS. 5 and 6 are flowcharts illustrating a processing procedure executed by the control section 51. First, the control section 51 determines whether or not the ion generator 2 is during operation (step S1). When the control section 51 determines that the ion generator 2 is not during operation (NO at step S1), the control section 51 determines whether or not the user has instructed to start an operation by pressing the operation switching button of the operation panel 18 (step S2). When the control section 51 determines that the ion generator 2 is during operation (YES at step S1), the control section 51 determines whether or not the user has instructed to switch the operation by pressing the operation switching button of the operation panel 18 (step S3).

When the user has instructed to start the operation at step S2 (YES at step S2), the control section 51 starts the operation of the air purifier 1 (step S4). The control section 51 executes step S8 described later after step S4. When the user has not instructed to start an operation at step S2 (NO at step S2), the control section 51 executes step S15 described later without starting the operation.

When the user has instructed to switch an operation at step 3 (YES at step S3), the control section 51 switches the operation (step S5). The operation is switched from "AUTOMATIC" to "CONTINUOUS", from "CONTINUOUS" to "INTERMITTENT", or from "INTERMITTENT" to "OFF."
It is noted that a method for switching the operation is not limited to the above-described example. For example, with the operation panel 18 including a buttons for "AUTOMATIC", "CONTINUOUS", "INTERMITTENT" and "OFF" respectively, the control section 51 may accept an instruction to switch the operation by the user's pressing these buttons.

Subsequently, the control section 51 determines whether or not the operation is "OFF" (step S6). When the operation is "OFF" (YES at step S6), the control section 51 stops the operation of the air purifier 1 (step S7). The control section 51 executes step S15 described later after step S7. When the operation is not "OFF" (NO at step S6), the control section 51 executes step S8 described later.

When the user has not instructed to switch the operation at step S3 (NO at step S3), the control section 51, without switching the operation, determines whether or not the user has instructed to switch the wind power by pressing the wind power switching button of the operation panel 18 (step S8). The control section 51 executes step S8 after step S4 or step S6 in the same manner.

When the user has instructed to switch the wind power (YES at step S8), the control section 51 switches the wind power to the instructed wind power (step S9). The wind power is switched from "HIGH" to "MEDIUM", from "MEDIUM" to "LOW", or from "LOW" to "HIGH."
It is noted that a method for switching the wind power is not limited to the above-described example. For example, with the operation panel 18 including buttons for "HIGH", "MEDIUM" and "LOW" respectively, the control section 51 may accept the instruction to switch the operation by the user's pressing these buttons.

The control section 51 executes step S10 described later after step S9. When the user has not instructed to switch the wind power (NO at step S8), the control section 51, without switching the wind power, determines whether or not the operation is "AUTOMATIC" (step S10).

When the operation is not "AUTOMATIC" (NO at step S10), the control section 51 determines whether or not the operation is "INTERMITTENT" (step S11). When the operation is "INTERMITTENT" (YES at step S11), the control section 51 executes step S14 described later. When the operation is not "INTERMITTENT" (NO at step S11), the control section 51 executes step S13 described later.

When the operation is "AUTOMATIC" (YES at step S10), the control section 51 determines whether or not the external power supply 58 supplies power to the air purifier 1 (step S12). It is noted that whether or not the external power supply 58 supplies power to the air purifier 1 is determined based on whether or not a voltage (of 5 volts, for example) is sensed at the sensing terminal of the control section 51 (see FIG. 4).

When the control section 51 determines that the external power supply 58 supplies power to the air purifier 1 (YES at step S12), the control section 51 causes the air purifier 1 to perform the continuous operation (step S13). The control section 51 executes step S15 described later after step S13. When the control section 51 determines that the external power supply 58 does not supply power to the air purifier 1 (NO at step S12), the control section 51 causes the air purifier 1 to perform the intermittent operation (step S14).
The control section 51 causes the ion generator drive circuit 53 and the fan motor drive circuit 54 to perform the continuous operation in the case where the external power supply 58 supplies power, and causes them to perform the intermittent operation in the case where the external power supply 58 does not supply power. Also, when the user has selected one of the "CONTINUOUS", "INTERMITTENT" and "OFF" operations at step S3, the control section 51 causes the ion generator drive circuit 53 and the fan motor drive circuit 54 to perform the operation selected by the user.

After the control section 51 causes the air purifier 1 to perform the continuous operation at step S13 or after the control section 51 causes the air purifier 1 to perform the intermittent operation at step S14, the control section 51 determines whether or not the external power supply 58 supplies power to the air purifier 1 again (step S15). When the control section 51 determines that the external power supply 58 supplies power to the air purifier 1 (YES at step S15), the control section 51 determines whether or not the internal power supply 65 is fully charged (step S16). For example, the control section 51 determines whether or not the internal power supply 65 is fully charged by sensing the voltage at the positive terminal of the internal power supply 65.
When the control section 51 determines that the external power supply 58 does not supply power to the air purifier 1 (NO at step S15), the control section 51 returns to step S1 and repeats the processing.

When the control section 51 determines that the internal power supply 65 is fully charged (YES at step S16), the control section 51 returns to step S1 and repeats the processing. When the control section 51 determines that the internal power supply 65 is not fully charged (NO at step S16), the control section 51 turns on the switch 66 (see FIG. 4) and applies the voltage of the external power supply 58 which is lowered by the step-down circuit 61 to the internal power supply 65 to charge the internal power supply 65 (step S17). After confirming that the internal power supply 65 is fully charged by sensing voltage at the positive terminal of the internal power supply 65, for example, the control section 51 turns off the switch 66, returns to step S1 and repeats the processing.

It is noted that when the air purifier 1 is not configured such that the internal power supply 65 is charged and does not include the switch 66, the control section 51 does not execute the processing from step S15 to step S17. The above-described situation is adapted to a case where the internal power supply 65 is taken out from the air purifier 1 and charged elsewhere, or in a case where the internal power supply 65 is replaced by a new one.

It is not always the case that the control section 51 causes the ion generator drive circuit 53 and the fan motor drive circuit 54 to perform the continuous operation in the case where the external power supply 58 supplies power, and to perform the intermittent operation in the case where the external power supply 58 does not supply power.
Switching the operation as described above is suitable for an environment such as the interior of a vehicle, where the air is easy to be contaminated in the case where the external power supply 58 supplies power and hard to be contaminated in the case where the external power supply 58 does not supply power. In the opposite environment where the air is hard to be contaminated in the case where the external power supply 58 supplies power and easy to be contaminated in the case where the external power supply 58 supplies power, however, the control section 51 may cause the ion generator drive circuit 53 and the fan motor drive circuit 54 to perform the intermittent operation in the case where the external power supply 58 supplies power and may cause them to perform the continuous operation in the case where the external power supply 58 does not supply power.

Moreover, the operations to be switched are not limited to the continuous operation and the intermittent operation. When the control section 51 determines that the external power supply 58 supplies power, the control section 51 may cause the air purifier 1 to perform the intermittent operation, and when the control section 51 determines that the external power supply 58 does not supply power, the control section 51 may cause the air purifier 1 to perform an intermittent operation with a duty ratio (a ratio of operating period to stopping period) less than a duty ratio of the intermittent operation performed in the case where the control section 51 determines that the external power supply 58 supplies power. This situation has a similar effect to that of the situation where the operation is switched between the continuous operation and the intermittent operation.

Furthermore, when the control section 51 determines that the external power supply 58 supplies power, the control section 51 may cause the air purifier 1 to perform the continuous operation (or the intermittent operation) and when the control section 51 determines that the external power supply 58 does not supply power, the control section 51 may cause the air purifier 1 to perform the continuous operation (or the intermittent operation), with lower power consumption than that of the continuous operation (or the intermittent operation) performed in the case where the control section 51 determines that the external power supply 58 supplies power. Since the air is cleaner by the purification of higher power consumption, this situation also has an effect similar to that of the situation where the operation is switched between the continuous operation and the intermittent operation. High or low power consumption may be realized by the wind power level or brightness of a light, for example.

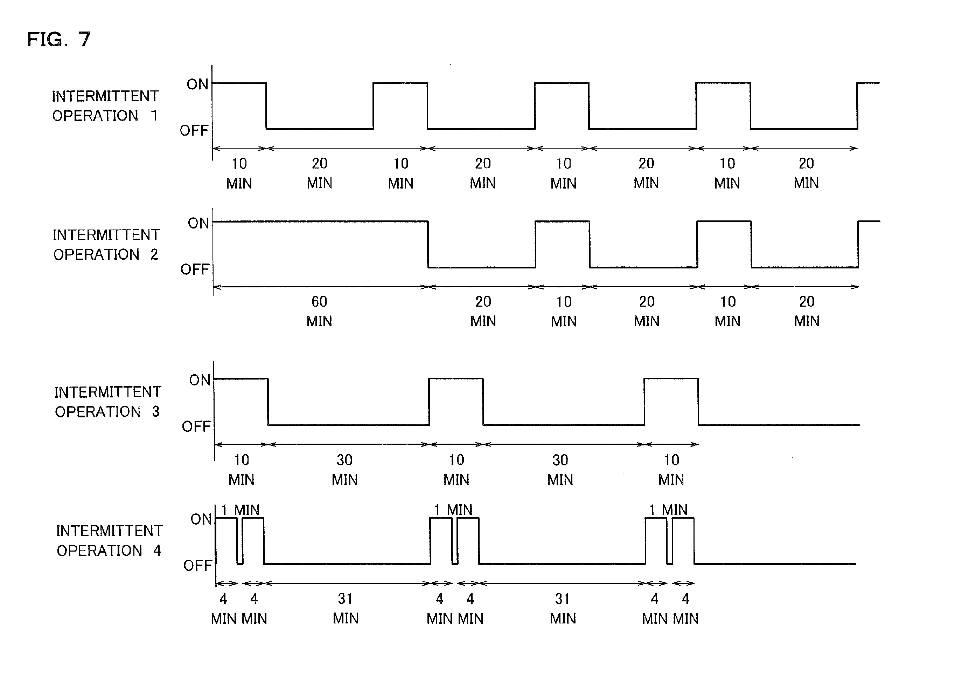

The intermittent operation performed by the control section 51 will be described below. FIG. 7 is a timing chart illustrating a timing of turning on/off of four types of intermittent operations. An intermittent operation 1 repeats 10 minutes of operating time and 20 minutes of stopping time (one cycle is 30 minutes). An intermittent operation 2 repeats 60 minutes of the operating time, 20 minutes of the stopping time, 10 minutes of operating time, 20 minutes of the stopping time, 10 minutes of the operating time and 20 minutes of the stopping time (one cycle is 140 minutes). An intermittent operation 3 repeats 10 minutes of the operating time and 30 minutes of the stopping time (one cycle is 40 minutes). An intermittent operation 4 repeats 4 minutes of the operating time, 1 minute of the stopping time, 4 minutes of the operating time and 31 minutes of the stopping time (one cycle is 40 minutes).

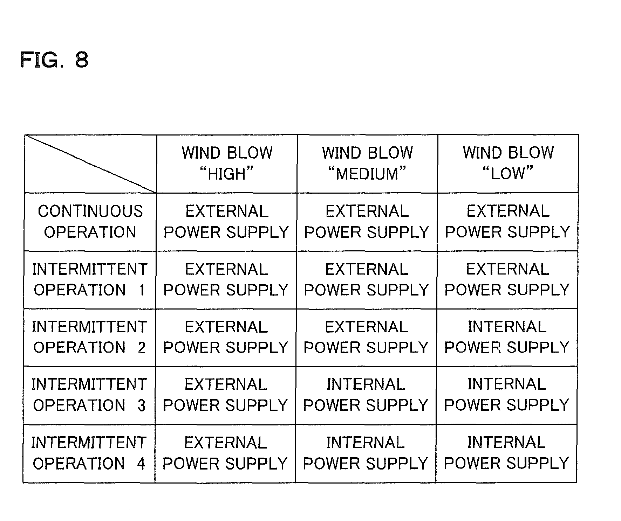

FIG. 8 is an illustrative table illustrating an example of a power supply used in accordance with each combination of contents of operation and wind power of an air blower 3. FIG. 8 illustrates the combination of the contents of operation and the wind power of the air blower 3 selected in a case where the operation is "AUTOMATIC." When the operation is not "AUTOMATIC," the air purifier 1 is operated in accordance with the contents of operation ("CONTINUOUS" or "INTERMITTENT") accepted from the user via the operation switching button provided on the operation panel 18. The air blow is set in accordance with the wind power setting accepted by the user. Therefore, the user judges how much the air is contaminated and can cause the air purifier 1 to perform the operation as he/she desires.

A case where the operation is "AUTOMATIC" will be described below. The continuous operation is performed independent from the wind blow of "HIGH," "MEDIUM" or "LOW" in the case where the external power supply 58 supplies power. In the case where the external power supply 58 does not supply power, the air purifier 1 will not be driven with the above-described operation and wind blow.

The intermittent operation 1, 2, 3 or 4 performed when the wind blow is "HIGH" is performed in the case where the external power supply 58 supplies power.
The "intermittent operation 1" and the "intermittent operation 2" with relatively high power consumption, performed when the wind blow is "MIDIUM" are performed in the case where the external power supply 58 supplies power. The "intermittent operation 3" and the "intermittent operation 4" with relatively low power consumption, performed when the wind blow is "MIDIUM" are performed in the case where the external power supply 58 does not supply power, that is, where only the internal power supply 58 supplies power.

The "intermittent operation 1" with relatively high power consumption, performed when the wind blow is "LOW" is performed in the case where the external power supply 58 supplies power. The intermittent operation 2, 3, or 4 performed when the wind blow is "LOW" is performed in the case where the external power supply 58 does not supply power, that is , where only the internal power supply 65 supplies power.

In the case where power is supplied to the air purifier 1 by the external power supply 58 such as the cigar lighter socket, the air purifier 1 is operated as illustrated in the flowcharts of FIGS. 5 and 6 by the internal power supply 65 when the external power supply 58 stops supplying power such as when the vehicle is parked. Here, the operation is switched from the continuous operation to the intermittent operation. More particularly, the operation is switched to any one of the intermittent operations 3 and 4 with the "MEDIUM" wind blow, and the intermittent operations 2 through 4 with the "LOW" wind blow.

In the case where the intermittent operation is performed with the "AUTOMATIC" operation, the operation performed when the internal power supply 65 supplies power may be set to, for example, the intermittent operation 4 with the "LOW" wind blow. Moreover, the operation for the internal power supply 65 may be switched to a combination with less power consumption every time when a predetermined time elapses. In the case where the external power supply 58 stops supplying power and is switched to the internal power supply 65, the control section 51 may cause the air purifier 1 to perform the intermittent operation 3 with the "MEDIUM" wind blow immediately after the switching, and to perform the intermittent operation 4 with the "LOW" wind blow after 30 minutes from the switching.

Furthermore, when the operation is not "AUTOMATIC," all the combinations illustrated in FIG. 8 may be made selectable. Only a part of the combinations of the operations illustrated in FIG. 8 may be made selectable considering that the user may make a mistake in operation or forget the operation.

Moreover, when the control section 51 may cause the air purifier to perform the intermittent operation with a great duty ratio in the case where the external power supply 58 supplies power, and to perform the intermittent operation with a small duty ratio in the case where the external power supply 58 does not supply power, the intermittent operation with a great duty ratio, for example, may be the intermittent operation 1 and the intermittent operation with the small duty ratio may be the intermittent operation 3.

Although the air purifier 1 used in an interior of a vehicle is explained in the present embodiment, a space where the air purifier 1 is adapted to is not limited the interior of the vehicle. At any places such as a hut which is equipped with a device that can be supplied with power to the air purifier 1 from the outside, effects similar to the above-described effects can be obtained by the air purifier 1.

Moreover, the air purifier adapted to the present invention is not limited to the air purifier 1 for purifying the air by generating ions. Any air purifiers that perform the operation for purifying the air by means of the internal power supply 65 and/or the external power supply 58 can be adapted to the present invention.

In the air purifier according to the present embodiment, the control section 51 determines whether or not the power is supplied by the external power supply 58 to cause the air purifier to perform the operation with different power consumption based on the determination. This enables the air to effectively be purified for a long period of time without increasing the capacity of the internal power supply 65 and the external power supply 58.
For example, in the interior of a vehicle, the continuous operation with the high power consumption may be performed in the case where the external power supply 58 supplies power, and the intermittent operation with the low power consumption may be performed in the case where the external power supply 58 does not supply power.
The external power supply 58 in the vehicle such as the cigar lighter socket is ready to supply power when the vehicle is ready to be driven. In the case where the external power supply 58 supplies power, it is more likely that the vehicle is occupied and the interior of the vehicle is easy to be contaminated. Meanwhile, in the case where the external power supply 58 does not supply power, it is less likely that the vehicle is occupied because the vehicle is not ready to be driven and thus the interior of the vehicle is hard to be contaminated. For this reason, the air can be purified effectively for a long period of time, if the continuous operation with high purification effect of the air and the high power consumption is performed when the interior is easy to be contaminated, and the intermittent operation with low purification effect of the air and the low power consumption is performed when the interior is hard to be contaminated.

In an air purifier according to the present embodiment, in the case where the external power supply 58 supplies power, the control section 51 turns on the switch 66 and the internal power supply 65 can be charged by the external power supply 58. This enables the air to be maintained in a condition purified by the internal power supply 65 for a longer time when the external power supply 58 stops supplying power.

In the air purifier according to the present embodiment, in the case where the external power supply 58 does not supply power, the intermittent operation is performed, and in the case where the external power supply 58 supplies power, the continuous operation or the intermittent operation with the duty ratio greater than the duty ratio of the intermittent operation performed in the case where the external power supply 58 supplies power is performed. This enables, as described above, the air to be purified more effectively for a longer time in an environment such as the interior of the vehicle where it is hard to be contaminated in the case where the external power supply 58 does not supply power and easy to be contaminated in the case where the external power supply 58 supplies power.

In the air purifier according to the present embodiment, in the case where the external power supply 58 supplies power, the operation with the low power consumption is performed, and in the case where the external power supply 58 supplies power, the operation with the high power consumption is performed. An example of the operation with low power consumption is a continuous operation with low wind power, and an example of the operation with the high power consumption is a continuous operation with high wind power. Here again, as the operation with the high power consumption purifies the air to be cleaner, the air can more effectively be kept clean for a longer period of time in an environment such as the interior of the vehicle.

In an air purifier according to the present embodiment, the control section 51 including the operation panel 18 causes the air purifier to perform the operation ("CONTINUOUS" or "INTERMITTENT") accepted from the user via the operation panel 18 when the operation is not "AUTOMATIC." For example, it is not always the case that the interior of the vehicle is not contaminated although the interior of the vehicle is hard to be contaminated in the case where the external power supply 58 does not supply power. Likewise, it is not always the case that the interior of the vehicle is contaminated although the interior of the vehicle is easy to be contaminated in the case where the external power supply 58 supplies power. Here, the user may check how much the air is contaminated and can cause the air purifier 1 to perform a more appropriate operation via the operation panel 18.

### Description of Reference Numerals

- 1: air purifier
- 18: operation panel (accepting section)
- 51: control section (control section and determining section)
- 58: external power supply
- 65: internal power supply

## Claims

1. An air purifier performing an operation for purifying air by means of an internal power supply and/or an external power supply, **characterized by** comprising:
a determining section for determining whether or not power is supplied by the external power supply; and
a control section for causing the air purifier to perform the operation with different power consumption based on a determination made by the determining section.

2. The air purifier according to claim 1,
wherein the internal power supply is chargeable by the external power supply.

3. The air purifier according to claim 1,
wherein the control section causes the air purifier to perform:
an intermittent operation in a case where power is not supplied by the external power supply; and
a continuous operation or an intermittent operation with a duty ratio greater than a duty ratio of the intermittent operation in a case where the power is supplied by the external power supply.

4. The air purifier according to claim 1,
wherein the control section causes the air purifier to perform:
an operation with low power consumption in a case where power is not supplied by the external power supply; and
an operation with high power consumption in a case where power is supplied by the external power supply.

5. The air purifier according to claim 1,
further comprising an accepting section for accepting a selection of either of a continuous operation and an intermittent operation,
wherein the control section causes the air purifier to perform an operation according to the selection accepted by the accepting section.
